# FASCICULE DE BREVET EUROPEEN

(11) **EP 0 690 041 B1**
(45) Date de publication et mention de la délivrance du brevet: **14.01.1998**
(21) Numéro de dépôt: 95401430.4
(22) Date de dépôt: 19.06.1995
(51) Int. Cl.: C07C 67/54, C07C 69/54, C07C 67/62, C07C 67/20

(54) **Procédé de fabrication de méthacrylate de méthyle sans diacétyle**
Verfahren zur Herstellung von Methylmethacrylat ohne Diacethyl
Process for the preparation of diacetyl free methylmethalcrylate

(30) Priorité: 30.06.1994 FR 9408103
(43) Date de publication de la demande: 03.01.1996
(73) Titulaire: ELF ATOCHEM S.A., 92800 Puteaux (FR)
(72) Inventeur: Croizy, Jean-François, F-75450 Farschivller (FR); Esch, Marc, F-57800 Freyming-Merlebach (FR)

(56) Documents cités:
- EP-A- 0 206 230

## Description

La présente invention porte sur un procédé de fabrication du méthacrylate de méthyle monomère par réaction d'acétone cyanhydrine avec de l'acide sulfurique pour former du méthacrylamide, lequel est ensuite estérifié par du méthanol.

Avec un tel procédé de fabrication, le méthacrylate de méthyle monomère est obtenu avec environ 1 à 5 ppm de diacétyle en tant qu'impureté responsable du jaunissement du poly(méthacrylate de méthyle). Pour éviter ce problème de jaunissement du polymère formé, il faut faire en sorte que la teneur en diacétyle dans le méthacrylate de méthyle monomère soit inférieure à 0,1 ppm.

Conformément au brevet européen EP-B-0 206 230, il est proposé d'ajouter au mélange réactionnel d'estérification une quantité efficace d'une ortho-diamine aromatique, telle que l'ortho-phénylène diamine, en présence d'un catalyseur acide (par exemple, l'acide sulfurique), lequel est présent en une quantité de 0,1 à 65% en poids du mélange réactionnel. L'ortho-diamine aromatique peut aussi être ajoutée après la séparation du monomère fini, avec addition d'une quantité appropriée d'acide minéral.

Un tel procédé permet une élimination satisfaisante du diacétyle ; cependant, il présente les inconvénients d'avoir à utiliser des ortho-diamines aromatiques qui sont des produits coûteux, toxiques, et qui peuvent entraîner une diminution de la stabilité du méthacrylate de méthyle monomère, du fait de la présence dans ce dernier du produit de réaction du diacétyle et de l'ortho-diamine aromatique. Ceci peut conduire à des encrassements fréquents au niveau des bouilleurs des colonnes de distillation.

La Société déposante a donc recherché une solution plus satisfaisante pour l'élimination du diacétyle dans le méthacrylate de méthyle monomère.

Il a maintenant été découvert de façon surprenante que l'utilisation d'une 1,2-diamine non aromatique permettait d'atteindre cet objectif.

La présente invention a donc pour objet un procédé de fabrication du méthacrylate de méthyle monomère, comprenant les étapes consistant à :
(a) faire réagir de l'acétone cyanhydrine avec de l'acide sulfurique ou un oléum, pour obtenir du méthacrylamide ;
(b) faire réagir le méthacrylamide ainsi obtenu soit directement, soit après l'avoir isolé, avec du méthanol en présence d'eau ;
(c) séparer par distillation le méthacrylate de méthyle du mélange acide sulfurique, sulfate d'ammonium et eau ;
(d) éliminer par extraction liquide-liquide le méthanol n'ayant pas réagi ;
(e) distiller le méthacrylate de méthyle lavé en deux stades :
   (e1) d'abord pour en éliminer les traces restantes d'eau, de méthanol et des impuretés à bas point d'ébullition ; puis
   (e2) pour en séparer le méthacrylate de méthyle pur des impuretés à haut point d'ébullition,
   caractérisé par le fait que l'on conduit l'étape (e) en présence d'au moins une 1,2-diamine non aromatique choisie parmi l'éthylène diamine ou le cis-diaminocyclohexane et introduite:
   - à raison de 1 à 200 moles par mole de diacétyle présent dans le méthacrylate de méthyle à distiller ; et
   - en un point tel que son temps de contact avec le méthacrylate de méthyle soit d'au moins 10 minutes, lui permettant de réagir de façon substantielle avec le diacétyle présent.

La réaction du diacétyle avec la 1,2-diamine non aromatique est catalysée par un acide, celui-ci étant l'acide méthacrylique présent dans le milieu en forte proportion (1 à 2% en poids) par rapport au diacétyle (quelques ppm). De même, on ne peut pas exclure quelques traces d'acide sulfurique dans le milieu.

Le temps de contact entre la 1,2-diamine non aromatique et le méthacrylate de méthyle à distiller est d'au moins 10 minutes. Il est, de préférence, de 10 à 30 minutes.

Par ailleurs, on introduit la 1,2-diamine non aromatique à raison de 1 à 200 moles, de préférence à raison d'au moins 10 moles, par mole de diacétyle présent dans le méthacrylate de méthyle à distiller.

De préférence, on introduit la 1,2-diamine non aromatique dans l'alimentation de la colonne de distillation de l'étape (e1), la distillation étant conduite en continu ou en discontinu.

La distillation de l'étape (e1) est généralement conduite sous pression réduite (par exemple, sous une pression de l'ordre de 0,4 bar). Quant à la distillation de l'étape (e2), elle est généralement conduite aussi sous pression réduite (par exemple, sous une pression d'environ 0,1 à 0,4 bar).

L'étape (e) est, par ailleurs, avantageusement conduite en présence d'au moins un inhibiteur de polymérisation, lequel est choisi notamment parmi la phénothiazine, le di-tertiobutylcatéchol, l'éther méthylique de l'hydroquinone, l'hydroquinone et leurs mélanges.

Le procédé conforme à la présente invention permet de conduire à un méthacrylate de méthyle ayant une teneur finale en diacétyle inférieure à 0,1 ppm (seuil de détection de la méthode d'analyse utilisée).

Les exemples suivants illustrent l'invention sans toutefois en limiter la portée. Dans ces exemples, les pourcentages sont indiqués en poids, sauf indication contraire.

### EXEMPLE 1 (Distillation en discontinu)

Dans une colonne à distiller, on introduit du méthacrylate de méthyle contenant 4,5 ppm de diacétyle, issu de l'étape (e1) d'un procédé conforme à la présente invention.

Dans le bouilleur, on injecte 5 ppm d'éthylène diamine. La distillation est conduite à 400 mbar pendant 2 heures environ. On récupère, en tête de colonne, du méthacrylate de méthyle, dont la teneur en diacétyle est mesurée par dosage U.V. de la quinoxaline obtenue par réaction du diacétyle restant sur de l'ortho-phénylène diamine rajoutée en excès en milieu acide.

Comme résultat, on obtient un méthacrylate de méthyle ne contenant plus que 0,1 ppm de diacétyle.

### EXEMPLE 2 (Distillation en continu) - étape (e2)

Dans une capacité à volume modulable, montée juste avant l'alimentation d'une colonne à distiller et maintenue à une température d'environ 75°C, on introduit du méthacrylate de méthyle contenant 2,3 ppm de diacétyle, issu de l'étape (e1) d'un procédé conforme à la présente invention, à raison de 300 cm³/h, ainsi qu'une solution de 1,2-éthylène diamine dans du méthacrylate de méthyle pur, à raison de 10 cm³/h. La concentration en 1,2-éthylène diamine est ajustée en fonction du rapport molaire voulu entre la 1,2-éthylène diamine et le diacétyle. Ce rapport est ici de 20.

Après un temps de séjour de 10 minutes dans cette capacité, on introduit le mélange au milieu de la colonne à distiller et on conduit une distillation sous une pression de 500 mBar, avec une température de bouilleur de 81 à 83°C et une température de tête de colonne de 54-55°C.

On récupère du méthacrylate de méthyle pur et des produits lourds, dans un rapport massique 88-90/12-10. Les produits lourds contiennent de la 1,2-éthylène diamine et la pyrazine formée.

Le méthacrylate de méthyle ainsi purifié contient moins de 0,1 ppm de diacétyle.

### EXEMPLES 3 à 7 - étape (e2)

On a répété l'Exemple 2 en modifiant le temps de séjour dans la capacité à volume modulable et le rapport molaire 1,2-éthylène diamine/diacétyle, comme indiqué dans le tableau ci-après.

Les résultats sont également rapportés dans le tableau ci-après.

Ces exemples, conduits sur un méthacrylate de méthyle issu de l'étape (e1), simulent une mise en contact du diacétyle avec la 1,2-éthylène diamine d'un méthacrylate de méthyle issu de l'étape (d), du fait qu'un temps de séjour existe dans le bouilleur de l'étape (e1).

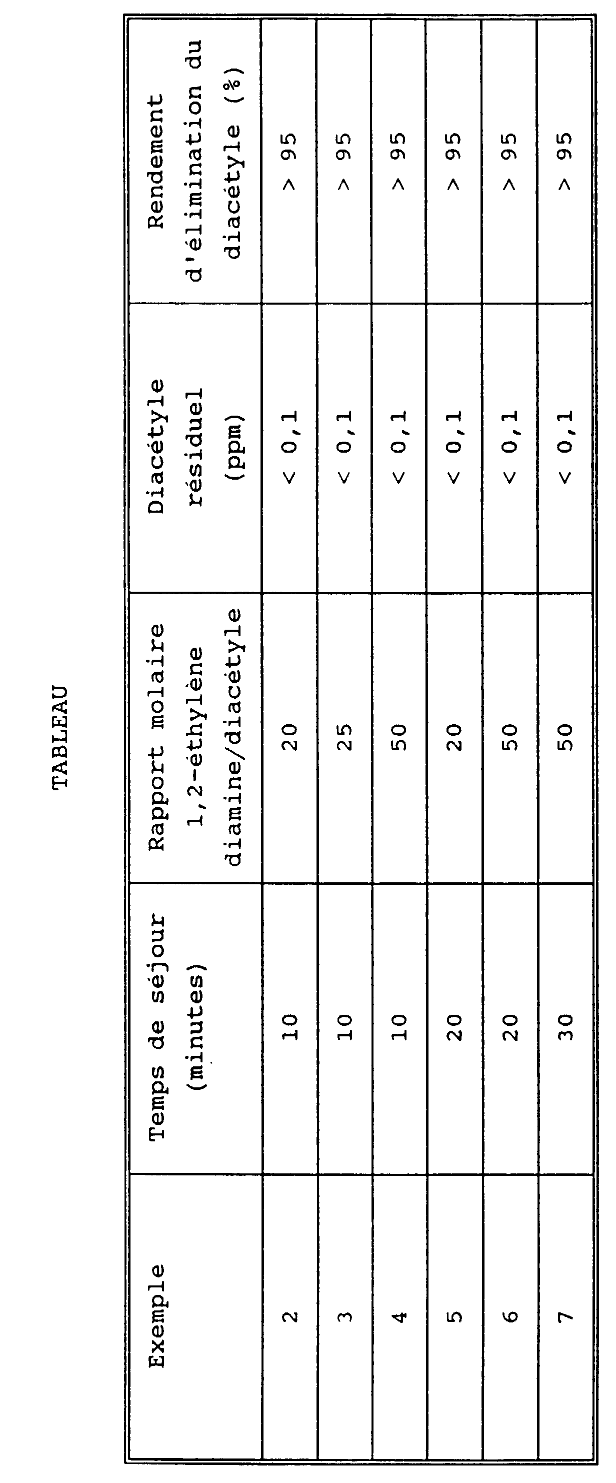

### EXEMPLE 8 - étape (e1)

Dans une colonne à distiller, en continu, on introduit, en milieu de colonne, du méthacrylate de méthyle contenant 3,5 ppm de diacétyle, issu de l'étape (d) d'un procédé conforme à la présente invention, à raison de 300 cm³/h, ainsi qu'une solution de 1,2-éthylène diamine dans du méthacrylate de méthyle pur, à raison de 10 cm³/h. La concentration en 1,2-éthylène diamine est ajustée en fonction du rapport molaire voulu entre la 1,2-éthylène diamine et le diacétyle. Ce rapport est ici de 200.

On conduit la distillation sous une pression de 500 mBar, avec une température de bouilleur de 81°C et une température de tête de colonne de 45°C.

En pied de colonne, on a récupéré du méthacrylate de méthyle et les produits lourds ; en tête de colonne, de l'eau et des produits légers dans un rapport massique 89,4 / 10,6. Après séparation du méthacrylate de méthyle et des produits lourds (étape (e2)), les produits lourds contiennent la 1,2-éthylène diamine et la pyrazine formée.

Le méthacrylate de méthyle ainsi purifié contient moins de 0,1 ppm de diacétyle.

Des essais successifs ont été réalisés sans nettoyage du dispositif utilisé. En fin de traitement, d'une durée totale d'environ 240 heures, aucun encrassement au niveau du bouilleur n'a été observé visuellement.

## Revendications

1. Procédé de fabrication du méthacrylate de méthyle monomère, comprenant les étapes consistant à :
(a) faire réagir de l'acétone cyanhydrine avec de l'acide sulfurique ou un oléum, pour obtenir du méthacrylamide ;
(b) faire réagir le méthacrylamide ainsi obtenu soit directement, soit après l'avoir isolé, avec du méthanol en présence d'eau ;
(c) séparer par distillation le méthacrylate de méthyle du mélange acide sulfurique, sulfate d'ammonium et eau ;
(d) éliminer par extraction liquide-liquide le méthanol n'ayant pas réagi ;
(e) distiller le méthacrylate de méthyle lavé en deux stades :
(e1) d'abord pour en éliminer les traces restantes d'eau, de méthanol et des impuretés à bas point d'ébullition ; puis
(e2) pour en séparer le méthacrylate de méthyle pur des impuretés à haut point d'ébullition,
caractérisé par le fait que l'on conduit l'étape (e) en présence d'au moins une 1,2-diamine non aromatique choisie parmi l'éthylène diamine ou le cis-diaminocyclohexane et introduite :
- à raison de 1 à 200 moles par mole de diacétyle présent dans le méthacrylate de méthyle à distiller ; et
- en un point tel que son temps de contact avec le méthacrylate de méthyle soit d'au moins 10 minutes, lui permettant de réagir de façon substantielle avec le diacétyle présent.

2. Procédé selon la revendication 1, caractérisé par le fait que l'on introduit la 1,2-diamine non aromatique à raison d'au moins 10 moles par mole de diacétyle présent dans le méthacrylate de méthyle à distiller.

3. Procédé selon l'une des revendications 1 et 2, caractérisé par le fait que le temps de contact entre la 1,2-diamine non aromatique et le méthacrylate de méthyle à distiller est de 10 à 30 minutes.

4. Procédé selon l'une des revendications 1 à 3, caractérisé par le fait que l'on introduit la 1,2-diamine non aromatique dans l'alimentation de la colonne de distillation de l'étape (e1), la distillation étant conduite en continu ou en discontinu.

5. Procédé selon l'une des revendications 1 à 4, caractérisé par le fait que la distillation de l'étape (el) est conduite sous pression réduite.

6. Procédé selon l'une des revendications 1 à 5, caractérisé par le fait que la distillation de l'étape (e2) est conduite sous pression réduite.

7. Procédé selon l'une des revendications 1 à 6, caractérisé par le fait que l'on conduit l'étape (e) en présence d'au moins un inhibiteur de polymérisation choisi notamment parmi la phénothiazine, le di-tertiobutylcatéchol, l'éther méthylique de l'hydroquinone, l'hydroquinone et leurs mélanges.

## Patentansprüche

1. Verfahren zur Herstellung von monomerem Methylmethacrylat mit folgenden Schritten:
(a) Umsetzung von Acetoncyanhydrin mit Schwefelsäure oder Oleum zur Herstellung von Methacrylamid;
(b) Umsetzung des so hergestellten Methacrylamids mit Methanol in Gegenwart von Wasser entweder direkt oder nach Abtrennung;
(c) Abtrennung des Methylmethacrylats von dem Gemisch aus Schwefelsäure, Ammoniumsulfat und Wasser durch Destillation;
(d) Abtrennung des unumgesetzten Methanols durch Flüssig-flüssig-Extraktion;
(e) Destillation des gewaschenen Methylmethacrylats in zwei Schritten:
(e1) zunächst Destillation zur Beseitigung der verbleibenden Spuren von Wasser, Methanol und Verunreinigungen mit niedrigem Siedepunkt; anschließend
(e2) Destillation zur Abtrennung des reinen Methylmethacrylats von Verunreinigungen mit hohem Siedepunkt,
dadurch gekennzeichnet, daß der Schritt (e) in Gegenwart mindestens eines nichtaromatischen 1,2-Diamins durchgeführt wird, das unter Ethylendiamin oder cis-Diaminocyclohexan ausgewählt ist und
- in Mengenanteilen von 1 bis 200 Mol pro Mol des in dem zu destillierenden Methylmethacrylat vorliegenden Diacetyls, und
- an einer Stelle zugegeben wird, daß die Kontaktzeit mit dem Methylmethacrylat mindestens 10 Minuten beträgt, wodurch es mit dem vorliegenden Diacetyl im wesentlichen Umgang reagieren kann.

2. Verfahren nach Anspruch 1, dadurch gekennzeichnet, daß das nichtaromatische 1,2-Diamin in Mengenanteilen von mindestens 10 Mol pro Mol des in dem zu destillierenden Methylmethacrylat vorliegenden Diacetyls zugegeben wird.

3. Verfahren nach einem der Ansprüche 1 und 2, dadurch gekennzeichnet, daß die Kontaktzeit des nichtaromatischen 1,2-Diamins mit dem zu destillierenden Methylmethacrylat 10 bis 30 Minuten beträgt.

4. Verfahren nach einem der Ansprüche 1 bis 3, dadurch gekennzeichnet, daß das nichtaromatische 1,2-Diamin in die Zufuhrleitung der Destillationskolonne des Schrittes (e1) gegeben wird, wobei die Destillation kontinuierlich oder diskontinuierlich geführt wird.

5. Verfahren nach einem der Ansprüche 1 bis 4, dadurch gekennzeichnet, daß die Destillation des Schrittes (el) unter vermindertem Druck durchgeführt wird.

6. Verfahren nach einem der Ansprüche 1 bis 5, dadurch gekennzeichnet, daß die Destillation des Schrittes (e2) unter vermindertem Druck durchgeführt wird.

7. Verfahren nach einem der Ansprüche 1 bis 6, dadurch gekennzeichnet, daß der Schritt (e) in Gegenwart mindestens eines Polymerisationsinhibitors durchgeführt wird, der insbesondere unter Phenothiazin, Di-tert.- butylbrenzcatechin, Hydrochinonmethylether, Hydrochinon und deren Gemischen ausgewählt ist.

## Claims

1. Process for the manufacture of monomeric methyl methacrylate, comprising the steps consisting in:
(a) reacting acetone cyanohydrin with sulphuric acid or an oleum, in order to obtain methacrylamide;
(b) reacting the methacrylamide thus obtained either directly, or after it has been isolated, with methanol in the presence of water;
(c) separating the methyl methacrylate from the sulphuric acid, ammonium sulphate and water mixture by distillation;
(d) removing the unreacted methanol by liquid-liquid extraction;
(e) distilling the washed methyl methacrylate in two stages:
(e1) firstly, in order to remove the remaining traces of water, of methanol and of low-boiling-point impurities; then
(e2) in order to separate the pure methyl methacrylate from the high-boiling-point impurities,
characterized in that step (e) is carried out in the presence of at least one non-aromatic 1,2-diamine which is chosen from ethylenediamine and cis-diaminocyclohexane and which is introduced:
- in an amount of 1 to 200 mol per mole of biacetyl present in the methyl methacrylate to be distilled; and
- at a point such that its contact time with the methyl methacrylate is at least 10 minutes, enabling it to react substantially with the biacetyl present.

2. Process according to Claim 1, characterized in that the non-aromatic 1,2-diamine is introduced in an amount of at least 10 mol per mole of biacetyl present in the methyl methacrylate to be distilled.

3. Process according to either of Claims 1 and 2, characterized in that the contact time between the non-aromatic 1,2-diamine and the methyl methacrylate to be distilled is from 10 to 30 minutes.

4. Process according to one of Claims 1 to 3, characterized in that the non-aromatic 1,2-diamine is introduced into the feed of the distillation column of step (e1), the distillation being carried out continuously or discontinuously.

5. Process according to one of Claims 1 to 4, characterized in that the distillation of step (el) is carried out at reduced pressure.

6. Process according to one of Claims 1 to 5, characterized in that the distillation of step (e2) is carried out at reduced pressure.

7. Process according to one of Claims 1 to 6, characterized in that step (e) is carried out in the presence of at least one polymerization inhibitor chosen, in particular, from phenothiazine, di-tert-butylcatechol, hydroquinone methyl ether, hydroquinone and mixtures thereof.
